(19) Europäisches Patentamt
European Patent Office
Office européen des brevets

(11) Veröffentlichungsnummer: **0 071 065**
**A1**

(12) # EUROPÄISCHE PATENTANMELDUNG

(21) Anmeldenummer: 82106208.0

(22) Anmeldetag: 12.07.82

(51) Int. Cl.³: C 07 C 51/58
C 07 C 63/70, C 07 C 65/21
C 07 C 53/50, C 07 C 45/41
C 07 C 47/14, C 07 C 47/55
C 07 C 47/575, B 01 J 27/10
B 01 J 23/44

(30) Priorität: 24.07.81 DE 3129273
24.07.81 DE 3129274

(43) Veröffentlichungstag der Anmeldung: 09.02.83 Patentblatt 83/6

(84) Benannte Vertragsstaaten: CH DE FR GB LI

(71) Anmelder: BAYER AG
Zentralbereich Patente, Marken und Lizenzen
D-5090 Leverkusen 1, Bayerwerk(DE)

(72) Erfinder: Braden, Rudolf, Dr.
Nothauserfeld 1
D-5068 Odenthal(DE)

(72) Erfinder: Klauke, Erich, Dr.
Eichendorffweg 8
D-5068 Odenthal(DE)

(54) **Verfahren zur Herstellung von durch Fluor substituierten Carbonsäurechloriden und deren Verwendung.**

(57) Durch Fluor substituierte Carbonsäurechloride können aus den entsprechenden durch Fluor substituierten Carbonsäurefluoriden durch Umsetzung mit Siliciumtetrachlorid oder Titantetrachlorid hergestellt werden.



Croydon Printing Company Ltd.

0071065

BAYER AKTIENGESELLSCHAFT
Zentralbereich
Patente, Marken und Lizenzen

5090 Leverkusen, Bayerwerk
Mn/bc/c

Verfahren zur Herstellung von durch Fluor substituierten Carbonsäurechloriden und deren Verwendung

Die Erfindung betrifft ein Verfahren zur Herstellung von fluorsubstituierten Carbonsäurechloriden und deren Verwendung für die Reduktion zu den entsprechenden durch Fluor substituierten Aldehyden.

Bei der Herstellung von durch Fluor substituierten Carbonsäurederivaten erfolgt die Einführung des Fluors in der Regel durch eine Austauschreaktion von Chlorgegen Fluoratome. Auf diese Weise kann das Fluor in aliphatische, aromatische und heterocyclische Carbonsäurederivate eingeführt werden. Zwangsläufig erhält man bei dieser Austauschreaktion auch das Carbonsäurefluorid (Houben-Weyl, Band V/3, Seiten 122 und 123 (1962)).

Bei der Absicht, aus dem so erhaltenen fluorierten Carbonsäurefluorid die sonst nur schwer zugänglichen entsprechenden Aldehyde durch Reduktion mit Wasserstoff in Gegenwart von Palladium nach der sogenannten Rosenmund-Reduktion herzustellen, ist es erforderlich,

die Carbonsäurefluoride in die entsprechenden Carbonsäurechloride umzuwandeln. Bei der Umwandlung tritt das Problem auf, daß das durch Austauschreaktion in den aliphatischen, aromatischen oder heterocyclischen Teil des Moleküls eingeführte Fluor ebenfalls wieder in unerwünschter Weise durch Chlor ersetzt wird.

Es ist bekannt, Carbonsäurefluoride in die Carbonsäurechloride in einer Zweistufenreaktion durchzuführen (Chemie u. Technologie aliph. fluororganischer Verbindungen, Seite 125, Enke Verlag 1964). Zuerst hydrolysiert man das Carbonsäurefluorid zu der Carbonsäure, die dann mit Thionylchlorid oder Phosphorchloriden in das entsprechende Carbonsäurechlorid überführt wird. Dieses Verfahren ist jedoch umständlich.

Aus der US 3 344 193 ist bekannt, aus fluorierten aliphatischen Dicarbonsäurechloriden durch Reduktion mit Wasserstoff in Gegenwart von Palladium als Katalysator nach der sogenannten Rosenmund-Reduktion die entsprechenden Aldehyde herzustellen. Bei dieser Reaktion ist es erforderlich, einen speziellen Palladium/Kohlenstoff-Katalysator einzusetzen, der kurz vorher für eine Hydrierung spezieller Säurechloride zu 2-Hydroxy-3,3,4,4,5,5-hexafluortetrahydropyran und 4-Hydroxy-2,2,3,3,4,4-hexafluorovaleriansäure benutzt worden ist (US 3 344 193, Spalte 3, Zeilen 34-39). Solche speziellen Katalysatoren für die Hydrierung von durch Fluor substituierten Carbonsäurechloriden sind sehr kompliziert und für den technischen Gebrauch kaum geeignet.

Die bekannten Katalysatorsysteme für die Rosenmund-Reduktion sind sehr empfindlich gegenüber Verunreinigungen, so daß die Selektivität und die Aktivität des Katalysators rasch abnehmen. Hierdurch ist ein hoher Katalysatorverbrauch bedingt.

Es wurde ein Verfahren zur Herstellung von durch Fluor substituierten Carbonsäurechloriden gefunden, das dadurch gekennzeichnet ist, daß man die entsprechenden durch Fluor substituierten Carbonsäurefluoride gegebenenfalls in Gegenwart eines Katalysators bei erhöhter Temperatur mit Siliciumtetrachlorid oder Titantetrachlorid umsetzt.

Die fluorsubstituierten Carbonsäurefluoride für das erfindungsgemäße Verfahren können Verbindungen der Formel (I)

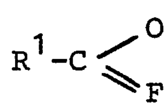

(I) ,

in der

$R^1$ einen durch Fluor substituierten aliphatischen, araliphatischen oder aromatischen Rest bedeutet,

sein.

Ein aliphatischer Rest kann erfindungsgemäß ein geradkettiger oder verzweigter Kohlenwasserstoffrest mit 1 bis 18, bevorzugt 1 bis 12, Kohlenstoffatomen sein. Ein besonders bevorzugter aliphatischer Rest ist der Niederalkylrest mit 1 bis etwa 6 Kohlenstoffatomen.

Die aliphatischen Reste sind durch Fluor substituiert. Im allgemeinen können die aliphatischen Reste 1 bis 3 Fluoratome enthalten. Bei längerkettigen aliphatischen Resten sind jedoch auch höher fluorierte, insbesondere perfluorierte, Alkylreste möglich. Beispielsweise seien die folgenden fluorierten aliphatischen Reste genannt: 3,3,3-Trifluorpropyl, Perfluorpropyl, Perfluorheptyl.

Ein aromatischer Rest ist im allgemeinen ein Arylrest, bevorzugt Phenyl, Biphenyl oder Naphthyl. Insbesondere bevorzugt ist der Phenylrest.

Die aromatischen Reste sind ebenfalls durch Fluor oder fluorhaltige Gruppen substituiert. Im allgemeinen enthalten die aromatischen Reste 1 bis 3 Fluoratome. Es sind jedoch auch höher fluorierte, im besonderen perfluorierte, aromatische Reste möglich.

Fluorhaltige Gruppen können außer Fluor noch zusätzlich andere Halogene wie Chlor enthalten. Beispielsweise seien hier Trifluormethyl, Difluor-chlormethyl und Fluor-dichlormethyl genannt.

Fluorhaltige Gruppen können auch die Trifluormethoxy- und Trifluormethylthioreste sein.

Die aromatischen Reste können außer durch Fluor noch durch andere Reste substituiert sein, die sich unter

den Reaktionsbedingungen nicht verändern. Beispielsweise seien hier niedere aliphatische Reste ($C_1$ bis etwa $C_6$) oder die Gruppen

$R^6-SO_2-$ oder

$R^6-\overset{O}{\overset{\|}{C}}-$

in denen

$R^6$ ein niederer Alkyl- ($C_2$ bis etwa $C_6$) oder Phenylrest bedeutet,

genannt.

Im besonderen werden für das erfindungsgemäße Verfahren durch Fluor substituierte Carbonsäurefluoride der Formel (II)

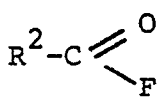

(II) ,

in der

$R^2$ ein durch 1 bis 3 Fluor substituierter niederer aliphatischer Rest oder ein aromatischer Rest

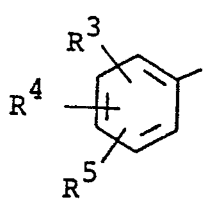

in dem
$R^3$, $R^4$ und $R^5$ gleich oder verschieden sind und Trifluormethoxy, Trifluormethylthio, Halogen oder einen durch 1 bis 3 Halogen substituierten oder unsubstituierten niederen aliphatischen Rest bedeuten, wobei wenigstens einer der Reste $R^3$, $R^4$ oder $R^5$ Fluor enthält, bedeuten oder für die Gruppen

$$R^6-SO_2- \text{ oder } R^6-\overset{O}{\overset{\|}{C}}- \quad ,$$

in denen
$R^6$ die obengenannte Bedeutung hat,

stehen,

bevorzugt.

Für das erfindungsgemäße Verfahren seien beispielsweise die folgenden fluorsubstituierten Carbonsäurefluoride genannt:

2-Trifluormethyl-, 3-Trifluormethyl-, 4-Trifluormethyl-, 4-Fluor-, 2-Fluor-, 2-Fluor-4-trifluormethyl-, 4-Trifluormethoxy-, 2-Fluor-3-chlor-, 4-Fluor-3-brom-,

3-Fluor-, 4-Fluor-3-phenoxy-, 4-Trifluormethyl-3-chlor-, 4-Phenylsulfonyl-3-trifluormethyl-, 3-Trifluormethyl-5-trifluormethoxy-benzoylfluorid, 3,3,3-Trifluorbuttersäurefluorid, α-Fluorphenylessigsäurefluorid, 3-Fluor-4-methylbenzoylfluorid, 3-Fluor-4-methoxybenzoylfluorid, 4-Fluorbenzoylessigsäurefluorid, 4-Fluorphenoxyessigsäurefluorid, 2-Fluorphenylessigsäurefluorid, 4-Fluorphenylessigsäurefluorid, α-Fluorzimtsäurefluorid, 4-Fluorbuttersäurefluorid, 2,4-Difluorbenzoylfluorid, 3,4-Difluorbenzoylfluorid, 2,5-Difluorbenzoylfluorid, 4-Fluorphenylbenzoylfluorid, Perfluorbuttersäurefluorid, Perfluorcaprylsäurefluorid.

Die erfindungsgemäße Herstellung von durch Fluor substituierten Carbonsäurechloriden wird in Gegenwart von Siliciumtetrachlorid oder Titantetrachlorid durchgeführt. Es kann vorteilhaft sein, im besonderen bei der Herstellung von durch Fluor substituierten aromatischen Carbonsäurechloriden, das erfindungsgemäße Verfahren zusätzlich in Gegenwart eines Chlorides der Elemente Aluminium, Titan, Antimon, Zinn oder Bor als Katalysator durchzuführen.

Bezogen auf das eingesetzte, durch Fluor substituierte Carbonsäurefluorid setzt man im allgemeinen 0,25 bis 0,375 Mol, bevorzugt 0,25 bis 0,3 Mol, Siliciumtetrachlorid oder Titantetrachlorid ein. Falls das erfindungsgemäße Verfahren zusätzlich in Gegenwart eines Katalysators durchgeführt wird, ist der Anteil des Katalysators im allgemeinen 0,1 bis 2 Gew.-%, bevorzugt 0,5 bis 1,2 Gew.-%, bezogen auf Siliciumtetrachlorid. Außer dem bevorzugten $AlCl_3$ können als Katalysatoren auch $TiCl_4$, $SbCl_5$, $SnCl_4$ oder $BCl_3$ eingesetzt werden.

Das erfindungsgemäße Verfahren wird im allgemeinen bei erhöhter Temperatur, bevorzugt im Temperaturbereich von 0 bis 250°C, durchgeführt. Im besonderen bevorzugt wird ein Temperaturbereich von 30 bis 230°C.

Im allgemeinen wird das erfindungsgemäße Verfahren unter Normaldruck durchgeführt; ein leichter Über- oder Unterdruck ist jedoch auch möglich (0,01 bar bis 5 bar).

Die Herstellung der durch Fluor substituierten Carbonsäurechloride kann beispielsweise wie folgt durchgeführt werden:

In einem Reaktionsgefäß werden das durch Fluor substituierte Carbonsäurefluorid und beispielsweise das Siliciumtetrachlorid gegebenenfalls mit dem Katalysator vorgelegt. Das Reaktionsgemisch wird erwärmt bis zur beginnenden Gasentwicklung. Das Reaktionsgemisch wird im erfindungsgemäßen Temperaturbereich gehalten, bis die Gasentwicklung beendet ist. Die Aufarbeitung des Reaktionsgemisches erfolgt durch Destillation.

Es ist überraschend, daß bei der Durchführung des erfindungsgemäßen Verfahrens das Fluor des Carbonsäurefluorids selektiv gegen Chlor ausgetauscht wird. Das Fluor, das an anderen Stellen des Moleküls gebunden ist, bleibt erhalten.

Die so erhaltenen durch Fluor substituierten Carbonsäurechloride können zur Herstellung von durch Fluor substituierten Aldehyden durch Reduktion mit Wasserstoff in Gegenwart eines für die Rosenmund-Reduktion üblichen Edelmetall Hydrier-Katalysators in einem Lösungsmittel verwendet werden.

Als Katalysatoren für das erfindungsgemäße Verfahren werden die für die Rosenmund-Reduktion üblichen Edelmetall Hydrier-Katalysatoren, insbesondere Palladium-Katalysatoren, verwendet. Das Edelmetall kann in feinverteilter Form oder auf einem Träger wie Kohle, Aluminiumoxid, Spinell oder Bariumsulfat aufgebracht

sein.

Der Katalysator kann im allgemeinen 0,5 bis 10 Gew.-%, bevorzugt 1 bis 6 Gew.-%, des Edelmetalls enthalten.

Als Lösungsmittel für die Reduktion sind alle Lösungsmittel geeignet, in denen die fluorhaltigen Säurechloride löslich sind und die weder durch Wasserstoff in Anwesenheit der Katalysatoren noch durch die Säurechloride angegriffen werden. Der Siedepunkt der Lösungsmittel kann in weiten Grenzen variieren. Bei Reduktion unter Druck können niedrig siedende Lösungsmittel verwendet werden. Geeignete Lösungsmittel sind Kohlenwasserstoffe und Ether. Als Beispiele seien Methylcyclohexan, fluorierte Kohlenwasserstoffe, Toluol, Xylole, Ethylbenzol, Tetralin, Tetrahydrofuran und Dioxan genannt.

Bei Verwendung der an sich für die Rosenmund-Reduktion üblichen Lösungsmittel setzt man vorteilhafterweise Regulatoren zu, durch die die Aktivität des Katalysators eingestellt wird. Als Regulatoren seien beispielsweise Chinolin-Schwefel, Thioharnstoff oder Formamide genannt. Sie werden in der Regel in einer Menge von 0,01 bis 1 Gew.-%, bezogen auf das eingesetzte Edelmetall, dem Reaktionsgemisch zugesetzt.

In einer bevorzugten Ausführungsform des erfindungsgemäßen Verfahrens verwendet man bei der Reduktion als Lösungsmittel Sulfone. Bei der Verwendung von Sulfonen als Lösungsmittel erübrigt sich überraschenderweise der Zusatz von Regulatoren.

Als Sulfone für das erfindungsgemäße Verfahren können Verbindungen der Formel (III)

$$R^7-SO_2-R^8 \qquad (III) \quad ,$$

in der

$R^7$ und $R^8$ gleich oder verschieden sind und Alkyl mit 1 bis 13 Kohlenstoffatomen, Aryl, Aralkyl, wobei der aliphatische Teil 1 bis 6 Kohlenstoffatome hat und Cycloalkyl mit 5 bis 7 Kohlenstoffatomen bedeuten oder wobei $R^7$ und $R^8$ über 4 bis 8 Kohlenwasserstoffglieder zu einer cyclischen Verbindung verbunden sein können,

eingesetzt werden.

Alkyl kann hier erfindungsgemäß ein geradkettiger oder verzweigter Kohlenwasserstoffrest mit 1 bis 13 Kohlenstoffatomen, bevorzugt einen Niederalkylrest (1 bis etwa 6 Kohlenstoffatome) sein. Beispielsweise seien die folgenden Alkylreste genannt: Methyl, Ethyl, Propyl, Isopropyl, Butyl, Isobutyl, Pentyl, Isopentyl, Hexyl und Isohexyl.

Aryl kann erfindungsgemäß Phenyl oder Naphthyl, bevorzugt Phenyl, sein.

Aralkyl kann erfindungsgemäß im aliphatischen Teil 1 bis etwa 6 Kohlenstoffatome, bevorzugt 1 bis etwa 2 Kohlenstoffatome, enthalten und im aromatischen Teil Phenyl oder Naphthyl, bevorzugt Phenyl, sein. Beispielsweise seien die folgenden Aralkylreste genannt: Benzyl, Ethylphenyl, Phenylethyl, 4-Methylbenzyl.

Cycloalkyl kann erfindungsgemäß 5 bis 7 Kohlenstoffatome, bevorzugt 5 oder 6 Kohlenstoffatome, enthalten.

Beispielsweise seien die folgenden Cycloalkylreste genannt: Cyclobutyl, Cyclopentyl, Cyclohexyl, Cycloheptyl und Cyclooctyl.

Es ist auch möglich, daß die beiden Substituenten der Sulfongruppe durch Kohlenwasserstoffglieder, bevorzugt Methylengruppen, zu einem cyclischen Sulfon verbunden sind.

Die genannten Reste können durch übliche Reste substituiert sein. Als mögliche Substituenten seien beispielsweise Niederalkyl (1 bis etwa 6 Kohlenstoffatome), Halogen wie Fluor, Chlor, und halogenierte Niederalkylgruppen genannt. Bevorzugt können die Alkylgruppen durch Fluor und/oder Chlor und die Arylreste durch Fluor, Chlor, Niederalkyl und/oder fluoriertes Niederalkyl substituiert sein.

Die Alkyl-, Aryl-, Aralkyl- und Cycloalkylreste können durch einen oder mehrere Reste, bevorzugt 1 bis 3 Reste, substituiert sein.

Beispielsweise seien die folgenden Sulfone genannt: Dimethylsulfon, Methylethylsulfon, Diisopropylsulfon, Dibutylsulfon, Di-ethylsulfon, Thiolan-S-dioxid, Diphenylsulfon, Phenylisobutylsulfon, Phenylethylsulfon, Phenylmethylsulfon, Phenylchlormethylsulfon, Phenyl-4-methylphenylsulfon, 4-Methylphenyl-ethylsulfon, 4-Methylphenyl-isopropylsulfon, Ethyl-benzylsulfon und 4,4'-Bis-chlorphenylsulfon.

Besonders bevorzugt wählt man für das erfindungsgemäße Verfahren ein Sulfon aus, das höher als der herzustellende Aldehyd siedet, so daß man nach der Reduktion den Aldehyd destillativ aus dem Reaktionsgemisch abtrennt und die verbleibende Suspension des Katalysators in dem Sulfon wieder zur Reduktion von neuem Säurechlorid verwenden kann.

Sulfone für das erfindungsgemäße Verfahren sind an sich bekannt. Sie können im allgemeinen in technischer Qualität eingesetzt werden. Das Sulfon für das erfindungsgemäße Verfahren soll praktisch kein Wasser enthalten.

In einer besonders bevorzugten Ausführungsform des erfindungsgemäßen Verfahrens behandelt man den Hydrierkatalysator vor oder während der Reduktion mit einer Mischung aus Kohlenmonoxid und Wasserstoff. Im allgemeinen behandelt man den Edelmetallhydrierkatalysator, beispielsweise ein Palladium-Auf-Träger-Hydrierkontakt, vor der Reduktion 5 bis 60 Minuten



BAD ORIGINAL

im Temperaturbereich von 20 bis 100°C in einem Wasserstoffstrom der bis zu 50 Vol.-% Kohlenmonoxid enthält. Es ist auch möglich, den Katalysator in dem Lösungsmittel, das bei der Reduktion verwendet wird, zu suspendieren und das Wasserstoff/Kohlenmonoxid-Gasgemisch durchzuleiten. Es ist ebenfalls möglich, die Kohlenmonoxid-Behandlung des Katalysators während der Reduktion durchzuführen, indem man dem Wasserstoff bis zu 10 Vol.-%, bevorzugt weniger als 1 Vol.-%, Kohlenmonoxid beimischt.

Das erfindungsgemäße Verfahren kann beispielsweise wie folgt durchgeführt werden:

Im allgemeinen werden der Katalysator und das Säurechlorid in dem Lösungsmittel gelöst und unter Durchleiten von Wasserstoff auf eine Temperatur im Bereich von 40 bis 180°C, bevorzugt von 80 bis 160°C, erhitzt, bis die Halogenwasserstoffentwicklung mindestens 90 %, bevorzugt 95 %, der Theorie erreicht hat. In der Regel ist die Reduktion in weniger als 5 Stunden beendet.

Es ist in der Regel vorteilhaft, wenn die Konzentration des Aldehyds im Lösungsmittel 50 Gew.-% nicht überschreitet. Bevorzugt wird die Reduktion so durchgeführt, daß eine Endkonzentration des Aldehyds im Lösungsmittel zwischen 15 und 35 Gew.-% erhalten wird.

Es kann auch vorteilhaft sein, den Katalysator zunächst im Lösungsmittel unter Durchleiten von Wasserstoff auf die Reaktionstemperatur zu erhitzen und dann das Carbonsäurechlorid zuzugeben.

Der Wasserstoffpartialdruck bei der Hydrierung soll wenigstens 0,5 bar betragen. Es kann vorteilhaft sein, bei 1 bis 3 bar Wasserstoffpartialdruck zu hydrieren. Es ist im allgemeinen nicht erforderlich, daß der Wasserstoffpartialdruck größer als 6 bar ist.

Überraschenderweise ist es mit Hilfe des erfindungsgemäßen Verfahrens möglich, aus durch Fluor substituierten Carbonsäurefluoriden, durch Fluor substituierte Aldehyde herzustellen. Diese Aldehyde sind sonst nur schwer zugänglich.

Durch Fluor substituierte Aldehyde können für Pharmazeutika und Pflanzenschutzmittel verwendet werden (DE-OS 22 10 687 und DE-OS 29 33 979).

Beispiel 1

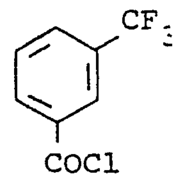

In einem Dreihalskolben mit Rührer und Rückflußkühler werden 1000 g 3-Trifluormethylbenzoylfluorid, 243 g $SiCl_4$ (= Mol 4:1,1) und 2 g $AlCl_3$ vorgelegt.

Es wird langsam hochgeheizt. Bei etwa 60°C setzt die Gasentwicklung ein. Im Maß der nachlassenden Gasentwicklung wird bis 160°C hochgeheizt (Dauer etwa 4 Stunden). Die Temperatur wird noch 1 Stunde bei 160 bis 170°C gehalten und dann der Ansatz destilliert. Man erhält 929 g 3-Trifluormethylbenzoylchlorid, Kp 77-8°/20 mbar, $n_D^{20}$ 1,4778.

Beispiel 2 (Verwendung)

In einem Dreihalskolben mit Rührer, Gaseinleitungsrohr und Rückflußkühler wurden 250 g Sulfolan (Thiolan-S-dioxid), 5 g Pd-$BaSO_4$ (Pd-Gehalt: 5 Gew.-%) und 100 g (0,48 Mol) 3-Trifluormethylbenzoylchlorid aus Beispiel 1 unter Durchleiten von Wasserstoff auf 140°C erhitzt. Die sofort einsetzende Chlorwasserstoffabspaltung war in 3 Stunden beendet. Ohne den Katalysator abzufiltrieren, wurde der gebildete 3-Trifluormethylbenzaldehyd abdestilliert.
$Kp_{20\ mbar}$ 69-70°C, $n_D^{20}$ 1,4648, 70 g;
Ausbeute: 84 % der Theorie.

Mit dem Rückstand der Destillation konnte noch mehrmals die Reduktion von je 100 g Säurechlorid in der gleichen Zeit durchgeführt werden.
Gesamtausbeute von 6 Ansätzen:
426 g 3-Trifluormethylbenzaldehyd, das sind 85,2 %.

Beispiel 3

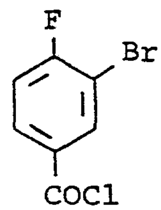

In einem Dreihalskolben mit Rührer und Rückflußkühler werden 958 g 3-Br-4-F-benzoylfluorid vom Kp 83°/15 mbar, F: 33°C, 184 g $SiCl_4$ (= Mol 4:1) und 1,8 g $AlCl_3$ vorgelegt und unter Rühren langsam hochgeheizt.

Bei etwa 30°C beginnt eine schwache Gasentwicklung, die ab 80°C recht zügig wird. Nach dem Abklingen wird langsam weiter geheizt bis 175°C. Nach 6 Stunden arbeitet man auf und destilliert. Nach einem Vorlauf von unverändertem Ausgangsmaterial das nochmals zur Chlorierung eingesetzt werden kann, erhält man 807 g 3-Brom-4-fluor-benzoylchlorid (78,4 % der Theorie) von 117°C/20 mbar, F: 42-44°C.

Beispiel 4 (Verwendung)

2 g Palladium/A-Kohle (5 Gew.-% Pd) und 350 g Toluol werden in einem Dreihalskolben mit Gaseinleitungsrohr, Rückflußkühler und Rührer 1 Stunde unter Durchleiten von Wassergas ($CO:H_2$ = 1) auf 75°C erhitzt. Dann wurden 70,0 g 3-Brom-4-fluorbenzoylchlorid, hergestellt nach Beispiel 3, zugegeben und anstelle von Wassergas wurde Wasserstoff eingeleitet. Die Temperatur wurde auf 85°C erhöht. Nach 4 Stunden waren 95 % der zu erwartenden Menge Chlorwasserstoff abgespalten worden. Das abgekühlte Reaktionsgemisch wurde filtriert und das Filtrat abdestilliert. Es wurden
53 g 3-Brom-4-fluorbenzaldehyd, $Kp_{20\ mbar}$ 112°C, $n_D^{30}$ 1,5685, F: 30°C erhalten.

Der Aldehyd zeigte im Gaschromatogramm eine Reinheit von 98 %. Ausbeute: 86,8 % der Theorie.

Der abfiltrierte Katalysator wurde für 3 weitere Reduktionen von je 70,0 g 3-Brom-4-fluorbenzoylchlorid verwendet. Es wurden die gleichen Ausbeuten erzielt wie im ersten Ansatz.

Beispiel 5 (Verwendung)

In einer Apparatur wie im Beispiel 2 wurden 100 g 4-Trifluormethylbenzoylchlorid, die analog Beispiel 1 hergestellt worden waren, in 700 ml trockenem Toluol mit 3,5 g Pd-Kohle (Pd-Gehalt 5 Gew.-%) in 4 Stunden bei 90°C hydriert; der Umsatz war 95 %. Der Pd-Kohle-Katalysator war zuvor 30 Minuten in Toluol bei Siedetemperatur mit einem Gemisch aus je 50 Vol.-% Wasserstoff und Kohlenmonoxid begast worden.
Durch Destillation konnten
69,2 g 4-Trifluormethylbenzaldehyd $Kp_{20\ mbar}$ 70-71°C, $n_D^{20}$ 1,4639, gewonnen werden. Ausbeute: 83 % der Theorie.

Beispiele 6 bis 10

Analog dem Verfahren in den Beispielen 1 und 2 wurden die in der folgenden Tabelle aufgeführten Säurechloride und Aldehyde hergestellt:

| Beispiele Nr. | R | Säurefluorid Ausgangsprodukt RCOF | Säurechlorid RCOCl | Aldehyd RCHO |
|---|---|---|---|---|
| 5 | 4-Trifluormethylphenyl | Kp 158-159° C<br>$n_D^{20}$ 1,4399 | $Kp_{23\ mbar}$ 82-83° C<br>$n_D^{20}$ 1,4760 | $Kp_{20\ mbar}$ 71° c<br>$n_D^{20}$ 1,4640 |
| 6 | 4-Trifluormethoxyphenyl | $Kp_{18\ mbar}$ 60-64° C<br>$n_D^{20}$ 1,4315 | $Kp_{16\ mbar}$ 85-86° C<br>$n_D^{20}$ 1,4740 | $Kp_{19\ mbar}$ 74° C<br>$n_D^{20}$ 1,4581 |
| 7 | 4-Fluorphenyl | $Kp_{19\ mbar}$ 51-53° C<br>$n_D^{20}$ 1,4792 | $Kp_{21\ mbar}$ 77° C<br>$n_D^{20}$ 1,5315 | $Kp_{16\ mbar}$ 65° C<br>$n_D^{20}$ 1,5211 |
| 8 | 2-Fluor-3-Chlorphenyl | $Kp_{10\ mbar}$ 83° C<br>Fp: 33° C | $Kp_{17\ mbar}$ 110° C<br>$n_D^{20}$ 1,5543 | $Kp_{15\ mbar}$ 84°C |
| 9 | 2,6-Difluor-phenyl | Kp 168° C<br>Fp: 36°C | Kp 191° C<br>$n_D^{20}$: 1,5010 | $Kp_{15\ mbar}$ 82-84°C<br>Fp: 17°C |
| 10 | 2,6-Fluor-chlor-phenyl | Kp: 79°C/16 mbar<br>$n_D^{20}$: 1,4970 | Kp: 90°C/17 mbar<br>$n_D^{20}$: 1,5271 | Kp: 104°C/20, mbar<br>Fp: 32-40°C |
| 11 | 3,3,3-Trifluorpropyl | Kp: 79°C<br>$n_D^{20}$: 1,3095 | Kp: 103°C<br>$n_D^{20}$: 1,3645 | Kp: 94-97°C<br>$n_D^{20}$: 1,3368 |



BAD ORIGINAL

Vergleichsbeispiel A

Das Beispiel 2 wurde wiederholt mit 100 g 3-Trifluormethylbenzoylchlorid, die mit Thionylchlorid aus der entsprechenden Säure gewonnen waren. Die Reduktion war erst nach 12 Stunden beendet, wie aus der Verfolgung der HCl-Abspaltung festzustellen war.

Die Ausbeute an 3-Trifluorbenzaldehyd betrug 76,6 % der Theorie. Mit dem Rückstand der Destillation konnte die Reduktion von weiteren 100 g Säurechlorid nicht in 15 Stunden zu Ende geführt werden. Die HCl-Abspaltung zeigte einen Umsatz von nur 76 % an.

Vergleichsbeispiel B

In einer Apparatur wie in Beispiel 2 wurden 100 g aus der Säure mittels Thionylchlorid hergestelltes 4-Trifluormethylbenzoylchlorid, 10 g eines 5 %igen Palladium-auf-Bariumsulfat-Katalysators, 1 ml einer 10 %igen Chinolin-Schwefel-Regulator-Lösung (vgl. Zymalkowski 1.c.) und 700 ml trockenes Xylol im Wasserstoffstrom auf Siedetemperatur erhitzt. Nach der Chlorwasserstoffabspaltung wurde ein Umsatz von 95 % nach 9,5 h erreicht.

Nach der Destillation im Vakuum wurden 60,2 g 4-Trifluormethylbenzaldehyd in einer Reinheit von 95,8 % erhalten, das entspricht einer Ausbeute von 69,1 %.

Patentansprüche

1) Verfahren zur Herstellung von durch Fluor substituierten Carbonsäurechloriden, dadurch gekennzeichnet, daß man die entsprechenden durch Fluor substituierten Carbonsäurefluoride gegebenenfalls in Gegenwart eines Katalysators bei erhöhter Temperatur mit Siliciumtetrachlorid oder Titantetrachlorid umsetzt.

2) Verfahren nach Anspruch 1, dadurch gekennzeichnet, daß man als Katalysator ein Chlorid der Elemente Aluminium, Titan, Antimon, Zinn oder Bor verwendet.

3) Verfahren nach den Ansprüchen 1 und 2, dadurch gekennzeichnet, daß man als Katalysator Aluminiumchlorid verwendet.

4) Verfahren nach den Ansprüchen 1 bis 3, dadurch gekennzeichnet, daß es im Temperaturbereich von 0 bis 250°C durchgeführt wird.

5) Verwendung von durch Fluor substituierten Carbonsäurechloriden nach Anspruch 1 zur Herstellung von durch Fluor substituierten Aldehyden durch Reduktion mit Wasserstoff in Gegenwart eines für die Rosenmund-Reduktion üblichen Edelmetall Hydrierkatalysators in einem Lösungsmittel.

6) Verwendung nach Anspruch 5, dadurch gekennzeichnet, daß Palladium als Katalysator eingesetzt wird.



BAD ORIGINAL

7) Verwendung nach Anspruch 5 und 6, dadurch gekennzeichnet, daß die Reduktion im Temperaturbereich von 80 bis 160°C durchgeführt wird.

8) Verwendung nach den Ansprüchen 5 und 6, dadurch gekennzeichnet, daß der Palladiumkatalysator mit einem Wasserstoff/Kohlenmonoxid-Gemisch behandelt wird.

9) Verwendung nach den Ansprüchen 5 bis 8, dadurch gekennzeichnet, daß die Reduktion in Gegenwart eines Sulfons als Lösungsmittel durchgeführt wird.

Europäisches Patentamt

# EUROPÄISCHER RECHERCHENBERICHT

0071065
Nummer der Anmeldung

EP 82106208.8

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | CH - A - 92 404 (DR.F. ZETZSCHE)<br>* Beispiele *<br>-- | 5,6 |
| A | DE - A1 - 2 506 157 (BUDAPESTI MÜSZAKI EGYETEM)<br>* Beispiele 1,2,9; Ansprüche *<br>-- | 5-7 |
| A | DE - B2 - 2 311 825 (TEIJIN)<br>* Spalten 5-8 *<br>-- | 1-4 |
| D,A | US - A - 3 344 193 (CARR et al.)<br>* Spalte 2, Zeilen 50-58; Spalte 3, Zeilen 34-39 *<br>---- | 5,6 |

KLASSIFIKATION DER ANMELDUNG (Int Cl.[3])

C 07 C 51/58
C 07 C 63/70
C 07 C 65/21
C 07 C 53/50
C 07 C 45/41
C 07 C 47/14
C 07 C 47/55
C 07 C 47/575
B 01 J 27/10
B 01 J 23/44

RECHERCHIERTE SACHGEBIETE (Int. Cl.[3])

C 07 C 45/00
C 07 C 47/00
C 07 C 51/00
C 07 C 53/00
C 07 C 63/00
C 07 C 65/00

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 25-10-1982 | HOFBAUER |

EPA form 1503.1 06.78